Europäisches Patentamt

European Patent Office

Office européen des brevets

(19)

(11) Veröffentlichungsnummer: **0 324 844 B1**

(12) **EUROPÄISCHE PATENTSCHRIFT**

(45) Veröffentlichungstag der Patentschrift: **23.12.92**

(51) Int. Cl.5: **A61B 17/22**

(21) Anmeldenummer: **88907094.2**

(22) Anmeldetag: **18.07.88**

(86) Internationale Anmeldenummer:
**PCT/DE88/00454**

(87) Internationale Veröffentlichungsnummer:
**WO 89/00408 (26.01.89 89/03)**

(54) **KATHETER ZUR ÜBERTRAGUNG VON LASERSTRAHLUNG.**

(30) Priorität: **16.07.87 DE 3723674**

(43) Veröffentlichungstag der Anmeldung:
**26.07.89 Patentblatt 89/30**

(45) Bekanntmachung des Hinweises auf die
Patenterteilung:
**23.12.92 Patentblatt 92/52**

(84) Benannte Vertragsstaaten:
**DE FR**

(56) Entgegenhaltungen:
**EP-A- 0 195 375**    **DE-A- 3 406 294**
**DE-A- 3 512 018**    **US-A- 3 995 623**
**US-A- 4 445 892**    **US-A- 4 672 961**
**US-E- 31 377**

(73) Patentinhaber: **BIOTRONIK Mess- und Therapiegeräte GmbH & Co Ingenieurbüro Berlin
Woermannkehre 1
W-1000 Berlin 47(DE)**

(72) Erfinder: **MÜLLER, Gerhard, J.
Krahmerstrasse 6-10
W-1000 Berlin 45(DE)**

(74) Vertreter: **Christiansen, Henning, Dipl.-Ing.
Patentanwalt CHRISTIANSEN Pacelliallee
43/45
W-1000 Berlin 33(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft einen Katheter der im Oberbegriff des Anspruchs 1 angegebenen Art.

Ein derartiger Katheter mit einem optischen Leiter, an dessen proximalem Ende eine Laser-Strahlungsquelle anschließbar ist und an dessen distalem Ende eine den Laserstrahl ablenkende Austrittsoptik angeordnet ist, ist aus der US-A-4 445 892 bekannt. Dabei handelt es sich um eine Kathetervorrichtung, die zweifach angeordnete Ballons aufweist, wobei durch ein Anschwellen der Ballons ein Blutgefäß verschlossen werden kann. Der bekannte Katheter weist eine Einrichtung zur Umlenkung des Laserstrahls gegenüber der axialen Erstreckung des optischen Leiters auf. Die Einrichtung zur Umlenkung des Laserstrahls besteht aus einer Reflexionsfläche, die im wesentlichen gegenüber der axialen Erstreckung des optischen Leiters geneigt angeordnet ist und den Laserstrahl asymmetrisch in eine Richtung ablenkt. Im Strahlengang des im wesentlichen um 90° umgelenkten Laserstrahles ist eine Austrittsöffnung vorgesehen.

Weitere Katheter mit einer entsprechenden Laser-Strahlungsquelle und einer Anordnung von optischen Elementen sind aus der DE-A-34 06 294, der EP-A-01 95 375 und der US-A-46 72 961 bekannt.

Eine häufige Komplikation im Kindesalter, aber auch bei Erwachsenen, ist das Auftreten von zusätzlichen Reizleitungsbahnen im Herzen, die den normalen Stimulationszyklus der Herzkontraktion in Systole und Diastole gravierend beeinträchtigen. Es ist bekannt, derartige zusätzliche Reizleitungsbahnen mittels elektrophysiologischer mono-, bi- oder multipolarer Meßkatheter zu diagnostizieren und dann gegebenenfalls über die Elektroden dieses Meßkatheters unter Anwendung hochfrequenter Ströme zu nekrotisieren, wobei jedoch die jeweils betroffene Nekrosezone sowie die Tiefe der Nekrotisierung unkontrolliert ist. Die Applikation hochfrequenter Ströme beeinträchtigt im hohen Grade auch nichtpathologische Leitungsbahnen und kann darüber hinaus generell das normale Herzrhythmusgeschehen stören. Dadurch ist eine elektrophysiologische Kontrolle des Zerstörungserfolges während der Koagulation durch die Applikation hochfrequenter Ströme nicht möglich.

Der vorliegenden Erfindung liegt die Aufgabe zugrunde, einen Katheter der eingangs genannten Art zu schaffen, mit dem bei geringen Strahlungsverlusten sowohl eine Untersuchung und/oder Therapie ermöglicht wird. Dabei soll eine elektrophysiologische Kontrolle des Zerstörungserfolges, d.h. der Nekrosezone und -tiefe bei einer Koagulation in pathologischen Bereichen eines Patienten ermöglicht werden.

Diese Aufgabe wird erfindungsgemäß durch das kennzeichnende Merkmal des Anspruchs 1 gelöst.

Die erfindungsgemäße Lösung ermöglicht insbesondere eine Koagulation subendokardial und subepikardial gelegener arrhythmogener Myokardstrukturen mit Hilfe eines kombinierten Elektroden/Laserstrahl-Katheters, wobei eine elektrophysiologische Kontrolle des zerstörungserfolges, d.h. der Nekrosezone und -tiefe während der Koagulation sichergestellt ist.

Vorteilhafte Ausgestaltungen der erfindungsgemäßen Lösung sind den Merkmalen der Unteransprüche zu entnehmen.

Die Erfindung soll nachstehend anhand mehrerer Ausführungsbeispiele näher erläutert werden. Es zeigen:

Figur 1 eine Ansicht eines kombinierten Elektroden/Laser-strahl-Herzkatheters mit Steuerelement und Austrittsoptik;

Figur 2 eine schematische Darstellung eines Glaszylinders als Austrittsoptik mit einer Einrichtung zur Ablenkung des Laserstrahles um 90°;

Figur 3 einen Schnitt durch ein kalibriertes Präzisions-Glas-Hüllrohr mit einer Laserstrahlablenkung von 90°;

Figur 4 eine Draufsicht auf das kalibrierte Präzisions-Glas-Hüllrohr gemäß Figur 3;

Figur 5 einen Schnitt durch einen bipolaren Katheterkopf mit einer Laserstrahlablenkung um 90° und

Figur 6 einen Schnitt durch den Katheterkopf gemäß Figur 5 entlang der Linie A-A.

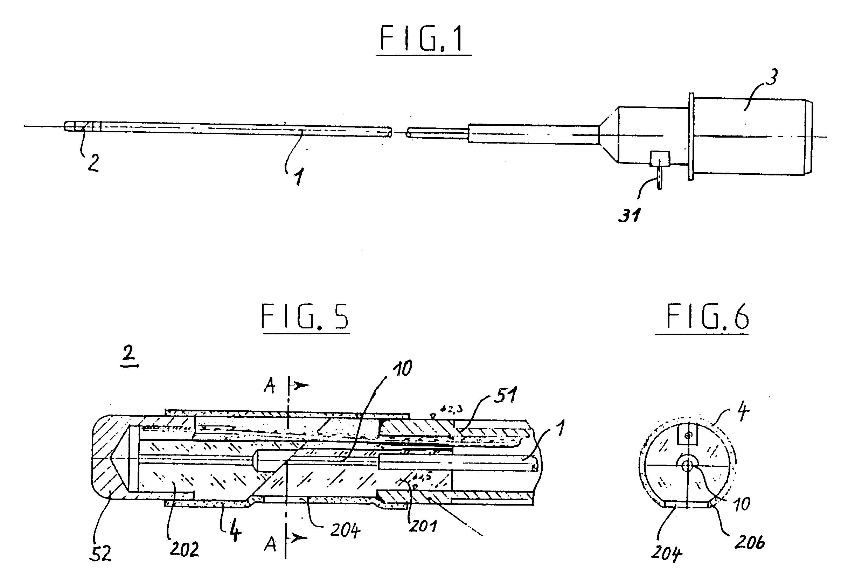

Der in Figur 1 dargestellte Herzkatheter weist an seinem distalen Ende einen bipolaren Katheterkopf 2 mit einer Austrittsoptik für Laserstrahlung auf, die über den Katheter 1 von einer nicht näher dargestellten Laser-Strahlungsquelle übertragen wird. Die Laserstrahlung wird am Steuerelement 3, das am proximalen Ende des Katheters 1 angeordnet ist, über eine Eintrittsöffnung 31 von der Laser-Strahlungsquelle eingekoppelt. Mit Hilfe des Steuerelementes 3 ist die Bewegung des Katheters 1 beim vortrieb durch die Gefäße eines Patienten bis zur Behandlungsstelle, im vorliegenden Falle insbesondere bis zur Stelle der Laserkoagulation, steuerbar.

Die Laserstrahlung wird über einen im Innern des Katheters 1 angeordneten optischen Leiter von der Eintrittsstelle 31 am Steuerelement 3 zur Austrittsoptik 2 am bipolaren Katheterkopf übertragen, wobei als optischer Leiter insbesondere eine einzelne Lichtleitfaser verwendet wird.

Nähere Einzelheiten der Ausgestaltung der Austrittsoptik im Katheterkopf 2 sind den nachfolgend beschriebenen Figuren zu entnehmen.

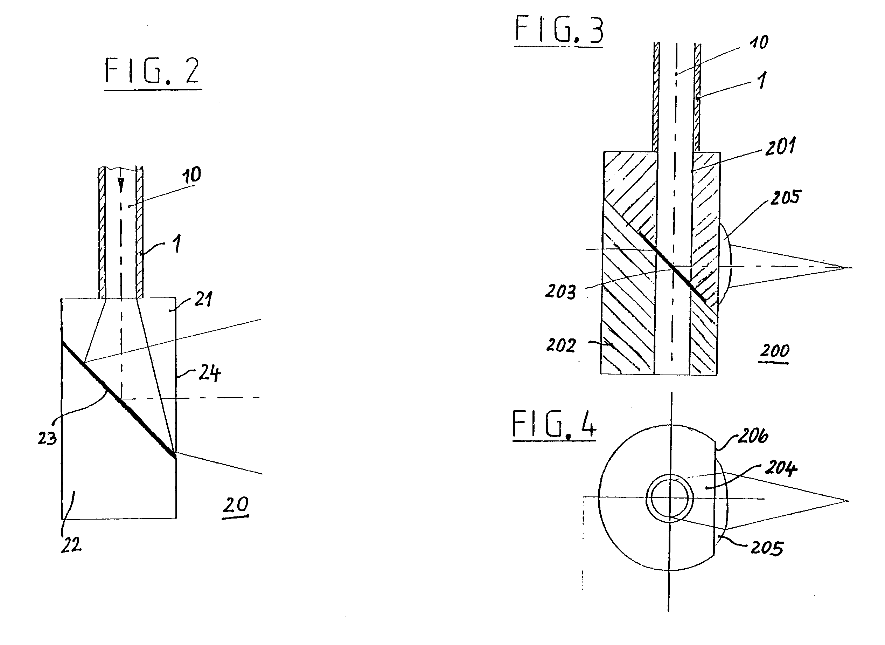

Figur 2 zeigt einen Längsschnitt durch eine als zylindrisches Endteil, insbesondere als Glaszylinder ausgebildete Austrittsoptik, bei der die im zentralen Kanal des Diagnosekatheters 1 angeordnete Lichtleitfaser 10 distal in Kontakt mit dem zweiteiligen Glaszylinder 20 steht, der mittig unter einem Winkel von 45° zur Bildung einer Reflektionsfläche 23 getrennt wurde. Die Schnittfläche ist poliert und beispielsweise durch Bedampfen verspiegelt, so daß eine Reflektionsfläche 23 mit hohem Reflektionsgrad gebildet wird. Nach dem Verspiegeln der Reflektionsfläche 23 werden die beiden Hälften 21, 22 des Glaszylinders wieder optisch zusammengefügt.

Dadurch entsteht ein zylindrisches Teil, das bei Beaufschlagung mit Laserstrahlung in axialer Richtung durch die Lichtleitfaser 10 mittig unter einem Winkel von 45° durch die Reflektions- oder Trennschicht 23, die Laserstrahlung unter 90° seitlich, d.h. asymmetrisch abstrahlt.

Um bei der Bewegung eines derartigen Katheters zum Behandlungsort insbesondere infolge der notwendigen Biegungen keine erhöhten Zugspannungen auf die Lichtleitfaser 10 auszuüben, kann die Lichtleitfaser 10 gemäß Figur 2b in axialer Richtung verschiebbar mit der Austrittsoptik verbunden werden. Da die axiale Verschiebung nur gering ist, genügt ein geringer Abstand a von ca. ¼ mm zwischen der Oberfläche des Glaszylinders 20 und dem distalen Ende der Lichtleitfaser 10.

In der in Figur 2 dargestellten Ausführungsform ist das distale Ende der Lichtleitfaser 10 in eine Bohrung in der Oberfläche des Glaszylinders 20 eingelassen. Selbstverständlich kann die Lichtleitfaser 10 gemäß Figur 2 auch auf die Oberfläche des Glaszylinders 20 im gestreckten Zustand des Katheters 1 aufgesetzt werden, so daß bei einer Krümmung des Katheters 1 die Lichtleitfaser 10 in der Katheterhülle gleiten und sich geringfügig von der Oberfläche des Glaszylinders 20 entfernen kann. Die mechanische Befestigung mit der Austrittsoptik erfolgt dabei über die Katheterhülle, was jedoch bei den geringfügigen mechanischen Beanspruchungen vollkommen ausreichend ist.

In Figur 3 ist eine Variante der erfindungsgemäßen Lösung bezüglich der Aufteilung der Austrittsoptik dargestellt. In dieser Ausführungsform ist die einzelne Lichtleitfaser direkt unter einem Winkel von 45° angeschliffen, wobei die in einem Kunststoffmantel des Katheters 1 angeordnete Lichtleitfaser 10 in die zentrale Bohrung eines kalibrierten Präzisions-Glas-Hüllrohres 200 eingeführt und darin verkittet ist. Nach der Verbindung der Lichtleitfaser 10 mit dem kalibrierten Präzisions-Glas-Hüllrohr 200 wird das Gesamtsystem des Hüllrohres 200 mit der Lichtleitfaser 10 mechanisch durchtrennt und unter einem Winkel von 45° angeschliffen. Anschließend wird auf den zentralen Teil der angeschliffenen Fläche mit der darin enthaltenen Lichtleitfaser 10 eine Spiegelschicht aufgedampft und anschließend die beiden Hälften 201, 202 des kalibrierten Präzisions-Glas-Hüllrohres 200 miteinander verklebt. Von der so gebildeten aufgedampften Spiegelschicht als Reflektionsfläche 203 wird die auftreffende Laserstrahlung um einen Winkel von 90° seitlich, d.h. asymmetrisch abgelenkt und kann gemäß Figur 4 aus einer Austrittsöffnung 204 austreten.

Zusätzlich kann gemäß Figur 4 das kalibrierte Präzisions-Glas-Hüllrohr 200 tangential bzw. radial angeschliffen werden, so daß die unter 90° austretende Laserstrahlung optisch nicht weiter beeinflußt wird. Ergänzend kann ein optisches Element 205 auf die tangential angeschliffene Fläche 206 aufgekittet werden, so daß die Laserstrahlung in geeigneter Weise fokussiert wird.

In den Figuren 5 und 6 ist im Längsschnitt bzw. im Querschnitt die spezielle Anwendung des Katheters als Herzkatheter zur Feststellung und Verödung zusätzlicher subendokardialer und subepikardialer arrhythmogener Myokardstrukturen durch Koagolation dieser Strukturen dargestellt.

Der in Figur 5 im Längsschnitt dargestellte Katheterkopf besteht aus einem kombinierten bipolaren Elektroden/Laserstrahl-Katheter mit einer Austrittsoptik, die beispielsweise gemäß Figur 3 aus einem kalibrierten Präzisions-Glas-Hüllrohr 200 besteht. Der Katheterkopf 2 ist mit dem Katheter 1 verbunden, wobei die im Katheter 1 enthaltene Lichtleitfaser 10 in eine zentrale Bohrung des Hüllrohres 200 eingeführt und mit der zentralen Bohrung verkittet ist. Nach dem Durchtrennen des Hüllrohres 200 in zwei Hälften 201 und 202, dem Anschleifen der Trennfläche unter einem Winkel von 45° und dem Bedampfen der Trennfläche mit einer Spiegelschicht werden die Hälften 201 und 202 wieder zusammengefügt und bilden eine Reflexionsfläche 203 zum Ablenken der über die Lichtleitfaser 10 geleiteten Laserstrahlung unter einem Winkel von 90°.

Der Katheterkopf 2 weist darüber hinaus zwei als Ringelektroden ausgebildete bipolare Elektroden 51, 52 auf, zwischen denen mittig die Austrittsöffnung 204 für die Laserstrahlung angeordnet ist.

Zweckmäßigerweise wird über die beiden Hälften 201, 202 der Austrittsoptik 200 sowie über jeweils einen Teil der bipolaren Ringelektroden 51, 52 eine Schutzhülle 4 in Form eines Schrumpfschlauches gezogen, der im Bereich der austretenden Laserstrahlung eine Austrittsöffnung 204 aufweist. Analog zur Ausgestaltung gemäß Figur 3 bzw. 4 ist das Hüllrohr 200 an dieser Stelle tangential angeschliffen, so daß die Möglichkeit besteht, die Laserstrahlung entweder unfokussiert oder mit Hilfe eines aufgekitteten optischen Elementes fokussiert aus der Austrittsoptik 200 austreten zu lassen. Die vorteilhafte Ausgestaltung der tangential angeschliffenen Fläche ist der Darstellung gemäß Figur 6, die einen Schnitt entlang der Linie A-A gemäß Figur 5 darstellt, zu entnehmen.

Durch Anwendung eines Nd:YAG-Lasers mit einer Wellenlänge der Laserstrahlung von 1,06 µm im Bereich von Leistungen unter 10 Watt bzw. eines Argon-Ionen-Lasers oder einer Halbleiter-Laserdiode, deren abgegebene Laserstrahlung über die Eintrittsöffnung 31 gemäß Figur 1 in einen Herzkatheter 1 eingekoppelt wird, können ausreichend tiefe Koagolationszonen im Myokard unter weitgehender Schonung der epikardialen und endokardialen Strukturen erzielt werden. Dabei ist die präzise und wiederholte Laseranwendung zur Ausschaltung, d.h. Verödung myokardialer Strukturen am besten durch eine Anwendung des sogenannten "touch-probe"-Verfahrens gewährleistet, wobei die Kombination von "touch-probe" mit zwei Elektrodenringen eines bipolaren Herzkatheters gemäß Figur 5 eine unmittelbare Laserbehandlung am Entstehungsort der Arrhythmie erlaubt. Damit ist eine perkutane transvenöse bzw. transarterielle Laserkoagulation subendokardial gelegener arrhythmogener Strukturen in den vier Herzkammern möglich.

Zur Schonung des Herzens wird die Abgabe der Laserstrahlung in die Ruhe- bzw. Refraktärphase des Herzens gelegt. Dies erfolgt mit Hilfe einer an ein EKG-Gerät angeschlossenen nicht dargestellten Synchronisationseinrichtung, die in der Refraktärphase ein Signal an die Laser-Strahlungsquelle zur Abgabe eines Laserstrahl-Impulses weiterleitet. Diese Synchronisationseinrichtung besteht aus einer Reihenschaltung folgender Komponenten: eine Detektionseinrichtung zur Ermittlung von spontanen oder stimulierten Herzaktionen, wie sie aus Herzschrittmacherschaltungen bekannt ist. An diese Detektionsschaltung schließt sich eine Torschaltung an, welche die Laseranordnung innerhalb eines der Refraktärzeit entsprechenden Zeitraums folgend auf die ermittelte Herzaktion zur Abgabe eines Laserimpulses freigibt. Die intermittierende Behandlung erfolgt damit automatisch ohne Zutun des behandelnden Arztes, der sich somit auf den Eingriff konzentrieren kann.

Dieses vorteilhafte, weil insbesondere hinsichtlich der Nekrosezone und -tiefe kontrollierbare, Verödungsverfahren bietet sich bei Patienten mit medikamentös therapierefraktären Rhythmusstörungen alternativ zur Rhythmuschirurgie und zur Behandlung mit antitachykarden Schrittmachern an.

Darüberhinaus besteht die Möglichkeit einer kausalen Therapie, die in Konkurrenz zur medikamentösen, rein symptomatischen Therapie steht. Die Verwendung kontrollierbarer Laserstrahlung ermöglicht ein präzises und nebenwirkungsfreies Veröden zusätzlicher Reizleitungsbahnen im Herzen und eine wesentliche Erweiterung der Indikation für Kathederkoagulationsverfahren. Damit wird die Voraussetzung einer ursächlichen Behandlung des WPW-Syndroms alternativ zu einer lebenslangen medikamentösen Behandlung chirurgisch durch intraoperatives epikardiales mapping und entsprechende Druchtrennung der Kent-Bündel ermöglicht.

Eine solche ursächliche Behandlung ist kausal und nicht nur symptomatisch und hat damit auf lange Sicht auch gegenüber einer lebenslangen medikamentösen Therapie volkswirtschaftliche Vorteile durch Reduktion der Kosten auf etwa ein Fünftel.

Neben der zuvor beschriebenen Anwendung des erfindungsgemäßen Katheters zur Feststellung und Verödung zusätzlicher Reizleitungsbahnen im Herzen sind weitere Anwendungsbereiche fokale oder durch Re-entry-Mechanismen hervorgerufene supraventrikuläre und vor allen Dingen auch ventrikuläre Tachykardien.

**Patentansprüche**

1. Katheter (1) mit einem optischen Leiter (10), an dessen proximalem Ende eine Laser-Strahlungsquelle anschließbar ist und an dessen distalem Ende eine den Laserstrahl ablenkende Austrittsoptik (2) angeordnet ist, die eine Einrichtung zur Umlenkung des Laserstrahls um im wesentlichen 90° gegenüber der axialen Erstreckung des optischen Leiters enthält, wobei die Einrichtung zur Umlenkung des Laserstrahls aus einer Reflexionsfläche (23,203) besteht, die vorzugweise um 45° gegenüber der axialen Erstreckung des optischen Leiters geneigt angeordnet ist und den Laserstrahl asymmetrisch in eine Richtung ablenkt, und einer im Strahlengang des um 90° umgelenkten Laserstrahles angeordneten Austrittsöffnung (204),

   **dadurch gekennzeichnet,**

   daß der Katheter (1) aus einem an eine Laser-Strahlungsquelle angeschlossenen bipolaren Diagnosekatheter zur Feststellung und Verödung zusätzlicher Reizleitungsbahnen im Herzen mit einem Katheterkopf (2) besteht, wobei die Austrittsöffnung für die um 90° umgelenkte Laserstrahlung im wesentlichen mittig zwischen den Elektroden (51, 52) des bipolaren Diagnosekatheters vorgesehen ist.

2. Katheter nach Anspruch 1, **dadurch gekennzeichnet,** daß die Austrittsoptik (20) aus einem zylindrischen Endteil besteht, das vorzugsweise mittig unter 45° getrennt, poliert und verspiegelt ist.

3. Katheter nach Anspruch 2, **dadurch gekenn-**

**zeichnet,** daß das distale Ende des optischen Leiters (10) axial verschiebbar mit der Austrittsoptik (20) verbunden ist.

4. Katheter nach Anspruch 1, **dadurch gekennzeichnet,** daß die Austrittsoptik (200) aus einem kalibrierten Präzisions-Glas-Hüllrohr besteht, in das der optische Leiter (10) eingekittet oder eingeklebt ist, daß das Hüllrohr (200) und der darin befindliche optische Leiter (10) unter einem Schnittwinkel von 45° gegenüber der axialen Erstreckung des optischen Leiters (10) getrennt und zur Bildung einer optischen Reflexionsfläche angeschliffen sind, und daß der abgetrennte Teil (202) des Hüllrohrs (200) mit dem darin befindlichen optischen Leiter (10) an den verbleibenden Teil (201) des Hüllrohrs (200) angekittet oder angeklebt ist.

5. Katheter nach Anspruch 4, **dadurch gekennzeichnet,** daß die zusammengefügten Teile (201, 202) des Hüllrohres (200) mit einer Schutzhülle (4), vorzugsweise mit einem Schrumpfschlauch, überzogen sind, die eine im Strahlengang des um 90° umgelenkten Laserstrahles angeordnete Austrittsöffnung (204) aufweist.

6. Katheter nach einem der vorangehenden Ansprüche, **dadurch gekennzeichnet,** daß der Bereich der Austrittsöffnung (24, 204) tangential angeschliffen ist.

7. Katheter nach Anspruch 6, **dadurch gekennzeichnet,** daß auf die tangential angeschliffene Fläche (206) ein die Laserstrahlung fokussierendes optisches Element (205) aufgekittet oder aufgeklebt ist.

8. Katheter nach einem der vorstehenden Ansprüche, **dadurch gekennzeichnet,** daß der optische Leiter (10) aus einer einzelnen Lichtleitfaser besteht.

9. Katheter nach einem der vorstehenden Ansprüche, **dadurch gekennzeichnet,** daß der Katheter steuerbar und am proximalen Ende des Katheters ein Steuerelement (3) angeordnet ist, das eine Eingangsöffnung (31) zur Einkopplung der Laserstrahlung aufweist.

10. Katheter nach einem der vorangehenden Ansprüche, **dadurch gekennzeichnet,** daß die mit dem proximalen Ende des Katheters verbundene Laser-Strahlungsquelle mit einer an ein EKG-Gerät angeschlossenen Synchronisationseinrichtung verbunden ist, die ein Signal zur Abgabe der Laserstrahlung bzw. eines Laserstrahl-Impulses in der Ruhephase bzw. Refraktärzeit des Herzens an die Laser-Strahlungsquelle abgibt.

11. Katheter nach einem der vorangehenden Ansprüche, **dadurch gekennzeichnet,** daß die Laser-Strahlungsquelle aus einem Nd:YAG-Laser, einem Argon-Laser oder einer Halbleiter-Laserdiode besteht.

12. Katheter nach einem der vorangehenden Ansprüche, **dadurch gekennzeichnet,** daß die Elektroden (51, 52) aus zwei Elektrodenringen bestehen, zwischen denen die Austrittsöffnung (204) für die Laserstrahlung vorgesehen ist.

## Claims

1. A catheter (1) with an optical conductor (10), to the proximal end of which a laser beam source can be connected and at the distal end of which there is arranged an exit optical system (2) which deflects the laser beam and which comprises a device for deflecting the laser beam by substantially 90° relative to the axial extent of the optical conductor, the device for deflecting the laser beam consisting of a reflection surface (23,203), which preferably is arranged inclined at 45° relative to the coaxial extent of the optical conductor and deflects the laser beam asymmetrically in one direction, and an exit opening (204) in the beam path of the laser beam deflected through 90°, characterised in that the catheter (1) consists of a bipolar diagnosis catheter, connected to a laser beam source, for the detection and obliteration of additional stimulation conduction paths in the heart, with a catheter head (2), the exit opening for the laser beam deflected through 90° being provided substantially centrally between the electrodes (51,52) of the bipolar diagnosis catheter.

2. A catheter according to claim 1, characterised in that the exit optical system (20) consists of a cylindrical end piece, which is preferably centrally split at 45°, polished and mirrored.

3. A catheter according to claim 2, characterised in that the distal end of the optical conductor (10) is connected axially displaceably to the exit optical system (20).

4. A catheter according to claim 1, characterised in that the exit optical system (200) consists of a calibrated precision-glass encasing tube, in which the optical conductor (10) is cemented or glued, in that the encasing tube (200) and the optical conductor (10) located therein are split at an angle of 45º relative to the axial extent of the optical conductor (10) and are ground to form an optical reflection surface, and in that the split part (220) of the encasing tube (200) with the optical conductor (10) located therein is cemented or glued to the remaining part (201) of the encasing tube (200).

5. A catheter according to claim 4, characterised in that the assembled parts (201,202) of the encasing tube (200) are covered with a protecting jacket (4), preferably with a shrunk sleeve, which has an exit opening (204) in the beam path of the laser beam deflected through 90º.

6. A catheter according to any one of the preceding claims, characterised in that the region of the exit opening (24,204) is ground tangentially.

7. A catheter according to claim 6, characterised in that to the tangentially ground surface (206) there is cemented or glued an optical element (205) which focuses the laser beam.

8. A catheter according to any one of the preceding claims, characterised in that the optical conductor (10) consists of a single optical fibre.

9. A catheter according to any one of the preceding claims, characterised in that the catheter is manoeuverable and there is arranged at the proximal end of the catheter a control element (3) which has an entry opening (31) for coupling in the laser beam.

10. A catheter according to any one of the preceding claims, characterised in that the laser beam source connected to the proximal end of the catheter is connected to a synchronisation device connected to an electrocardiogram apparatus, the synchronisation device providing a signal to the laser beam source for producing the laser beam or a laser beam pulse in the quiescent phase or refractory period of the heart.

11. A catheter according to any of the preceding claims, characterised in that the laser beam source consists of a Nd: YAG-laser, an argon laser or a semiconductor laser.

12. A catheter according to any one of the preceding claims, characterised in that the electrodes (51,52) consist of two electrode rings, between which there is provided the exit opening (204) for the laser beam.

## Revendications

1. Cathéter (1) comportant un guide optique (10) à l'extrémité proximale duquel peut être raccordée une source de rayonnement laser et à l'extrémité distale duquel est disposée une optique de sortie (2) déviant le faisceau laser et contenant un dispositif de coudage du faisceau laser selon sensiblement 90° par rapport à l'extension axiale du guide optique, le dispositif de coudage du faisceau laser étant composé d'une surface réfléchissante (23, 203) inclinée de préférence à 45° par rapport à l'extension axiale du guide optique et qui dévie asymétriquement le rayon laser dans un sens, et d'un orifice de sortie (204) disposé dans la marche des rayons du faisceau laser coudé à 90°, caractérisé en ce que

le cathéter (1) est composé d'un cathéter de diagnostic bipolaire relié à une source de rayonnement laser en vue de la détermination et de la destruction de voies additionnelles de conduction de l'excitation cardiaque, comportant une tête (2) de cathéter, l'orifice de sortie du rayonnement laser coudé à 90° étant prévu sensiblement centralement entre les électrodes (51, 52) du cathéter de diagnostic bipolaire.

2. Cathéter selon la revendication 1, caractérisé en ce que l'optique de sortie (20) est composée d'une partie terminale cylindrique qui est de préférence divisée centralement à 45°, polie et recouverte d'une couche réfléchissante.

3. Cathéter selon la revendication 2, caractérisé en ce que l'extrémité distale du guide optique (10) est reliée axialement, de façon mobile, à l'optique de sortie (20).

4. Cathéter selon la revendication 1, caractérisé en ce que l'optique de sortie (200) se compose d'un tube de précision gainant en verre calibré dans lequel le guide optique (10) est mastiqué ou collé, en ce que le tube gainant (200) et le guide optique (10) qui s'y trouve sont divisés selon un angle d'intersection de 45° par rapport à l'extension axiale du guide optique (10) et meulés en vue de la formation d'une surface réfléchissante optique et en ce que la partie séparée (202) du tube gainant (200) ainsi que le guide optique (10) qui s'y trouve est mastiquée ou collée à la partie restante (201) du tube gainant (200).

**5.** Cathéter selon la revendication 4, caractérisé en ce qu'est enfilée sur les parties réunies (201,202) du tube gainant (200) une gaine protectrice (4), de préférence un tube thermorétrécissable qui présente un orifice de sortie (204) disposé dans la marche des rayons du faisceau laser coudé à 90°.

**6.** Cathéter selon une des revendications précédentes, caractérisé en ce que la zone de l'orifice de sortie (24,204) est meulée tangentiellement.

**7.** Cathéter selon la revendication 6, caractérisé en ce qu'un élément optique (205) focalisant le rayonnement laser est mastiqué ou collé sur la surface (206) meulée tangentiellement.

**8.** Cathéter selon l'une des revendications précédentes, caractérisé en ce que le guide optique (10) est composé d'une seule fibre optique.

**9.** Cathéter selon l'une des revendications précédentes, caractérisé en ce que le cathéter peut être commandé et en ce qu'est disposé à l'extrémité proximale du cathéter un élément de commande (3) qui présente un orifice d'entrée (31) en vue de l'introduction du rayonnement laser.

**10.** Cathéter selon l'une des revendications précédentes, caractérisé en ce que la source de rayonnement laser reliée à l'extrémité proximale du cathéter est reliée à un dispositif de synchronisation raccordé à un appareil d'électrocardiographie, lequel dispositif émet un signal en vue de l'émission du rayonnement laser ou d'une impulsion du rayonnement laser durant la phase de repos ou la période réfractaire du coeur à l'intention de la source de rayonnement laser.

**11.** Cathéter selon l'une des revendications précédentes, caractérisé en ce que la source de rayonnement laser est composée d'un laser Nd : YAG, d'un laser à argon ou d'une diode laser à semiconducteur.

**12.** Cathéter selon l'une des revendications précédentes, caractérisé en ce que les électrodes (51, 52) sont composées de deux électrodes annulaires entre lesquelles est prévu l'orifice de sortie (204) du rayonnement laser.

FIG.1

FIG.5

FIG.6

EP 0 324 844 B1

FIG.3

FIG.4

FIG.2